# EUROPEAN PATENT APPLICATION

(11) **EP 0 002 925 A1**
(43) Date of publication of application: **11.07.1979**
(21) Application number: 78300853.5
(22) Date of filing: 18.12.1978
(51) Int. Cl.: C07D 213/64, A01N 9/22, C07B 19/00

(54) **Preparation of herbicidal 2-(4-(pyridyl-2-oxy)-phenoxy)- propionic acid derivatives and compounds thus obtained**

(30) Priority: 23.12.1977 GB 5375377
(71) Applicant: IMPERIAL CHEMICAL INDUSTRIES PLC, London SW1P 3JF (GB)
(72) Inventor: Cartwright, David, Reading Berkshire (GB)
(74) Representative: Downes, John Edward

(57) **Abstract**

The dextrorotatory optical isomer of the acid of formula: 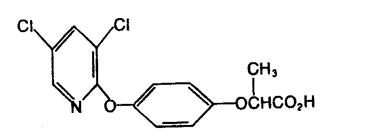 and salts, esters and other derivatives thereof, useful as selective herbicides for grass weeds in broad-leaved crops, and having more favourable herbicidal properties then the DL racemic form of the acid.

## Description

This invention relates to herbicidal pyridine compounds to processes for preparing them, and to herbicidal processes and compositions utilising them.

United States Patent No 4046553 discloses, inter alia, that pyridine compounds of the formula (I) below: 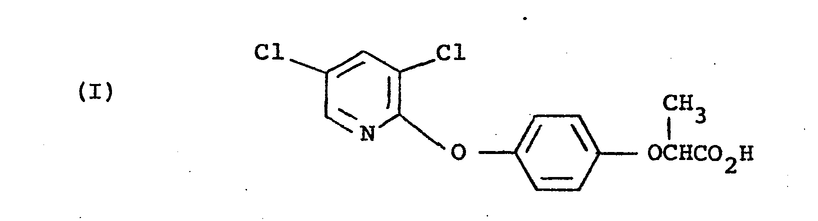 and salts, esters, and other derivatives thereof, are herbicides useful for selective control of grass weeds growing among broad-leaved crop plants.

The carbon atom adjacent to the carboxyl group in the above formula (I) is an asymmetric carbon atom. Accordingly, it is possible for the molecule to exist in two optical isomers (designated as the "D" and "L" forms). When prepared by chemical synthesis in the ordinary way, for example by the methods disclosed in U.S. Patent 4046553, the compound (I) will be obtained as a racemic mixture of equal proportions of the D and L optical isomers.

A method has now been devised for separating the D and L forms of compound (I) above, and it has been discovered that the D isomer possesses more favourable herbicidal properties than the racemic mixture of D and L forms of the acid (I). It has also been established that the D form of the acid (I) is the dextrorotatory isomer.

The D form of compound (I) may be converted by standard methods well known in the art to its corresponding salts, and ester and other derivatives.

In addition to the method of obtaining the D optical isomer of compound (I) above, which comprises separating this isomer from the DL racemic mixture, the D form of compound (I) above and its derivatives may also be obtained by stereospecific synthesis, for example by reacting 3,5-dichloro-2-p-hydroxyphenoxypyridine with the appropriate optical isomer of a-bromo or a-chloropropionic acid or a derivative thereof, in the presence of a base, as described for the corresponding racemic compounds in column 11, lines 1 to 47 of U.S. Patent 4046553.

Thus, the methyl ester of the D form of compound (I) may be prepared by reaction of 3,5-dichloro-2-p-hydroxy- phenoxypyridine with the methyl ester of L a-bromo or a-chloropropionic acid in the presence of a base, the propionic acid part of the molecule undergoing inversion from the L to the D configuration during the reaction. The optical purity of the compound (I) or its derivative prepared in this way will depend upon the optical purity of the a-bromopropionic or a-chloropropionic acid derivative used as starting material. Thus a starting material comprising 90% of the L form of methyl a-bromopropionate will not provide a product containing more than 90% of the D form of the methyl ester of compound (I).

The starting material should preferably contain as near 100% of the L isomer of the a-bromo or a-chloropropionic acid derivative as possible, so as to obtain a product which approaches as closely as possible to the herbicidal properties of the pure D isomer of compound (I) and its derivatives. However, it may be convenient to use a starting material which comprises somewhat less than 100% L isomer, although this will give rise to a product with less than the herbicidal effect of the pure D isomer of compound (I) or its derivatives.

According to the present invention there are provided herbicidal compounds having the structural formula (II): 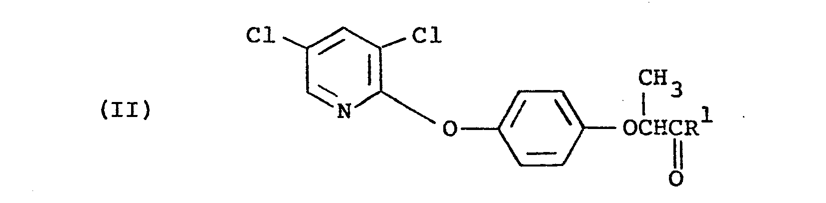 wherein more than 50% by weight of the compound is in the D optical isomer form, and wherein R¹ stands for a hydroxy group; an OM group wherein M is a cation; a straight or branched chain alkoxy group of 1 to 9 carbon atoms, optionally substituted by one or more halogen or hydroxy groups; an alkenyloxy group of 2 to 4 carbon atoms; a cyclohexyloxy group optionally substituted by one or more halogen atoms or methyl groups; a phenoxy group optionally substituted by one or more halogen atoms or methyl groups; a benzyloxy group optionally substituted in the phenyl moiety with one or more halogen atoms or methyl groups; an amino group optionally substituted with one or more alkyl groups containing 1 to 4 carbon atoms; an anilino group in which the phenyl moiety may be substituted by one or more halogen atoms or methyl groups; a morpholino group; or a piperidino group. Examples of cations include metal cations, for example sodium and potassium; and ammonium and substituted ammonium cations, for example mono-, di-, tri- and tetra-alkyl ammonium cations in which the alkyl groups contain from 1 to 4 carbon atoms. Preferably the compounds contain at least 75% by weight of the D optical isomer, and more preferably 90%. Still more preferably, the proportion of the D optical isomer is at least 99% by weight. A particularly preferred compound according to the invention is the compound of formula II wherein R¹ is a butoxy group.

In a further aspect the invention provides a process of controlling graminaceous weeds which comprises applying to the weeds a herbicidally effective amount of the compound of formula (II) above. More particularly, the invention provides a process of selectively controlling graminaceous weeds in the presence of broad-leaved crops which comprises applying to the crop area a compound of the formula (II) as hereinbefore defined, in an amount sufficient to inhibit the growth of the graminaceous weeds but insufficient to damage the crop substantially.

The rate at which the compounds will be applied in the process of the invention will depend upon factors such as the identity of the particular graminaceous weeds and broad-leaved crop, but in general an amount of from 0.025 to 2.5 kilograms per hectare will be suitable, while from 0.1 to 1 kilogram per hectare is preferred.

In a further aspect, the invention provides herbicidal compositions, comprising as an active ingredient the compound of formula (II) as hereinbefore defined in admixture with a carrier comprising a solid or liquid diluent.

The invention is illustrated in the following Examples.

### EXAMPLE 1

This Example illustrates the separation of the "D" optical isomer from a racemic mixture of the D and L optical isomers of 2[4(3,5-dichloropyridyl-2-oxy)-phenoxy]propionic acid (Compound (I) above).

A solution of l(-)-a-methylbenzylamine (12.12 g) was added to a solution of the racemic mixture of isomers of compound (I) (32.8 g) in ethanol (150 ml) with stirring. The solution was evaporated in a vacuum and the residue crystallised from toluene to give a white solid (13.8 g) of melting point 148°C. This was recrystallised twice from toluene to give the l(-)-a-methylbenzylamine salt (7.1 g) of melting point 156°C. The free acid was obtained from the salt by taking it up in water, acidifying, extracting the acidic solution with ether, drying the ether extracts, and evaporating the ether to give the acid, having a melting point of 122°C. The acid was found to be dextrorotatory when examined in solution in a polarimeter.

A sample of the D acid so obtained was converted to its methyl ester by heating under reflux for 1 hour with methanol containing p-toluenesulphonic acid. The reaction mixture was concentrated, diluted with ether, and shaken with sodium bicarbonate solution. The ether extracts were dried and evaporated to yield the methyl ester as an oil.

A sample of the methyl ester so obtained was dissolved in deuterochloroform and its nuclear magnetic resonance (NMR) spectrum examined. A singlet peak attributable to the ester methyl group was observed in the spectrum. Upon addition of an optical shift reagent (praseodymium tris[D-heptafluorobutyroyl-camphorate]), the peak attributed to the ester methyl group remained as a singlet peak, indicating that only a single optical isomer was present. The limits of accuracy of the observations were such that the dextrorotatory isomer was at least 95% pure; that is to say, less than 5% of the laevorotatory isomer was present.

For comparison, a solution of the racemic (DL) form of the methyl ester of compound (I) was examined. A single peak attributed to the ester methyl group was observed. Upon addition of the optical shift reagent referred to above, the singlet peak attributed to the ester methyl split into two separate peaks, corresponding to the two optical isomers present. -

### EXAMPLE 2

This Example illustrates the stereospecific synthesis of the D form of the methyl ester of compound (I) containing a minor proportion of the L form.

3,5-Dichloro-2-(4-hydroxyphenoxy)pyridine (0.2 g) and methyl a-bromo propionate (0.1 g of a mixture of 83% by weight of the L form and 17% by weight of the D form) were heated and stirred under reflux in methyl ethyl ketone (5 ml) containing anhydrous potassium carbonate (O.2 g) for 4k hours. The mixture was cooled, filtered, and the residue washed with methyl ethyl ketone. The filtrate was evaporated to leave an oil. The oil was purified by thin layer chromatography on silica gel using a plate 20 x 20 cm with a 2 mm layer of silica gel, and chloroform'as the solvent. The purified oil solidified on storage. Examination by nuclear magnetic resonance spectroscopy using an optical shift reagent as described in Example 1 showed that the product contained 75% of the D form of the methyl ester of compound (I) and 25% of the L form. Higher proportions of the D form can be obtained by using starting material containing a higher proportion of the L form of methyl a-bromo propionate. The L form of methyl a-bromo propionate may be obtained essentially free from the D form, for example by synthesis from L glycine as described by W L F Armarego, B A Milloy and W Pendergast, J. Chem. Soc., Perkins Transactions I, (1976), 2229.

### EXAMPLE 3

This Example illustrates the preparation of the methyl ester of the D form of compound (I), using as starting material the D form of compound (I) itself.

The D form of compound (I) (0.05 g) was heated under reflux in methanol (10 ml) with a trace of p-toluenesulphonic acid for 3 hours. The solvent was removed and the residue taken up in chloroform and washed with sodium bicarbonate solution, and the chloroform solution dried and evaporated. Examination by nuclear magnetic resonance spectroscopy showed the presence of 99.1% by weight of the D form of the methyl ester of compound (I). The butyl ester was prepared in a similar way by heating the D form of compound (I) with butanol and a trace of p-toluenesulphonic acid at 100°C for 4 hours, and isolating the butyl ester as described above for the methyl ester. A sample of the butyl ester was converted to the methyl ester by heating with excess of methanol in the presence of a trace of p-toluenesulphonic acid. Examination of the nuclear magnetic resonance spectrum of this ester showed the presence of 99.33% by weight of the D form of the methyl ester. The small amounts of the L form in the esters described above result from the presence of a small amount of the L form of compound (I) in the starting material.

### EXAMPLE 4

This Example illustrates the herbicidal properties of optical isomers according to the invention, and derivatives thereof.

Aqueous dispersions of test compounds were sprayed on a variety of growing plants (post-emergence) at a variety of rates. Results after 26 days scored on a scale from 0 (no herbicidal effect) to 9 (complete kill) are shown in Table 2 below. -

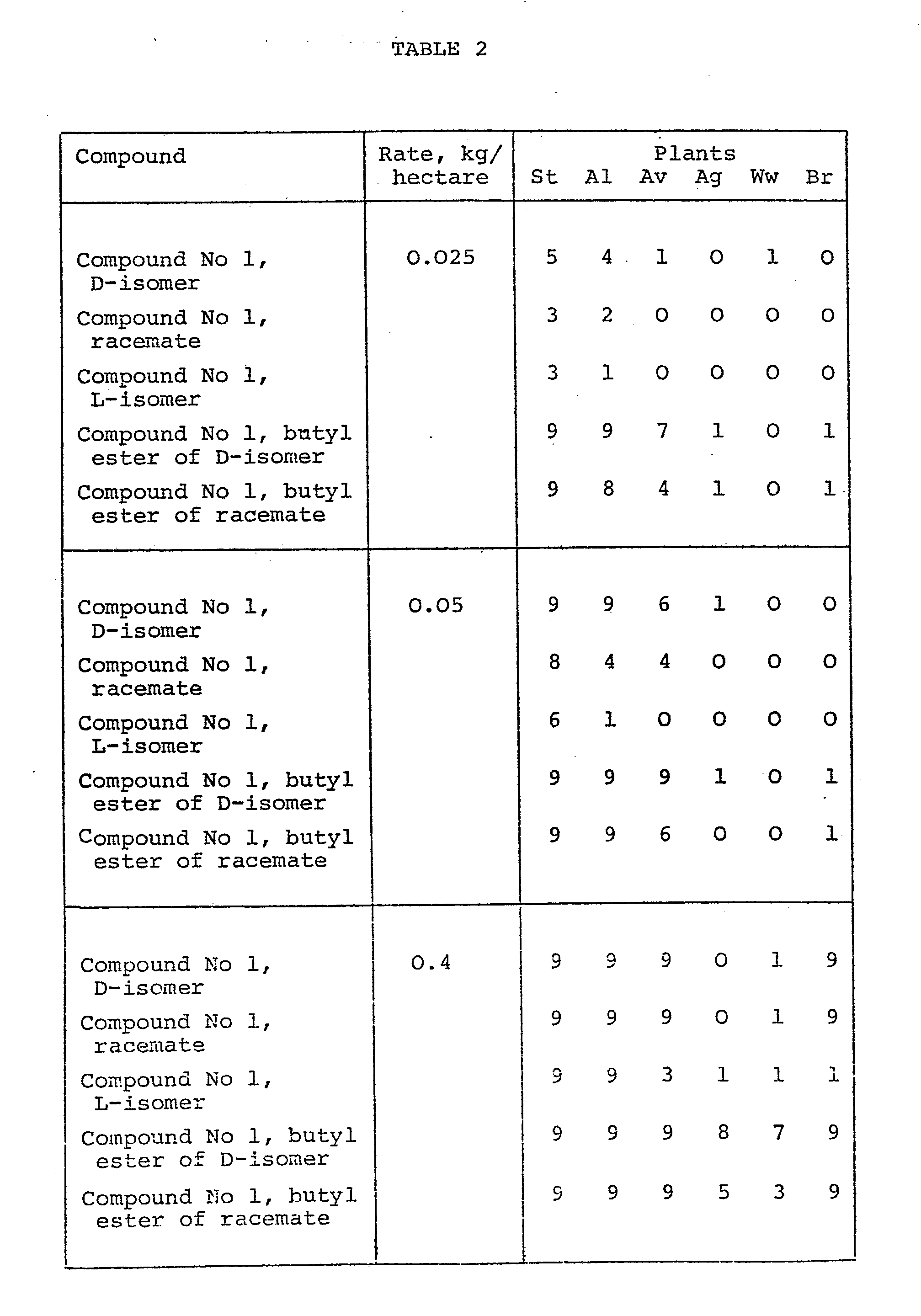

Similar tests were conducted in which fibre trays of soil were sown with seeds of the test plants and then sprayed with the compounds. These tests showed substantially no differences between the racemates and the resolved isomers, perhaps indicating that the optically active compound racemises in contact with the soil.

The plant species used in the test were:
St = Setaria viridis
Al = Alopecurus myosuroides
Av = Avena fatua
Ag = Agropyron repens
Ww = Winter wheat
Br = Barley

## Claims

1. Herbicidal compounds having the structural formula (II) 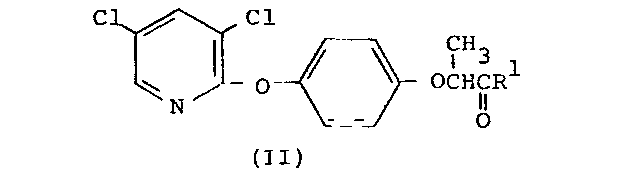 wherein more than 50% by weight of the compound is in the D optical isomer form, and wherein R¹ stands for a hydroxy group; an OM group wherein M is a cation; a straight or branched chain alkoxy group of 1 to 9 carbon atoms, optionally substituted by one or more halogen or hydroxy groups; an alkenyloxy group of 2 to 4 carbon atoms; a cyclohexyloxy group optionally substituted by one or more halogen atoms or methyl groups; a phenoxy group optionally substituted by one or more halogen atoms or methyl groups; a benzyloxy group optionally substituted in the phenyl moiety with one or more halogen atoms or methyl groups; an amino group optionally substituted with one or more alkyl groups containing 1 to 4 carbon atoms; an anilino group in which the phenyl moiety may be substituted by one or more halogen atoms or methyl groups; a morpholino group; or a piperidino group.

2. Herbicidal compounds as claimed in claim 1, wherein the proportion of the D optical isomer form is at least 75%.

3. Herbicidal compounds as claimed in claim 1 wherein the proportion of the D optical isomer form is at least 90%.

4. Herbicidal compounds as claimed in claim 1 wherein the proportion of the D optical isomer form is at least 99%.

5. A herbicidal compound as claimed in claims 1 to 4 wherein the group R¹ is a n-butoxy group.

6. Herbicidal compositions comprising as an active ingredient a compound as claimed in any of the preceding claims, in admixture with a solid or liquid diluent.

7. A process of controlling graminaceous weeds, which comprises applying to the weeds a herbicidally effective amount of a compound as claimed in any of claims 1 to 4.

8. A process of selectively controlling graminaceous weeds, in the presence of a broad-leafed crop, which comprises applying to the area of the growing crop a compound as claimed in any of claims 1 to 4 in an amount sufficient to inhibit the growth of the graminaceous weeds but insufficient to damage the crop substantially.

9. A process as claimed in claim 7 wherein the rate of application of the compound is from 0.025 to 2.5 kilograms per hectare.

10. A process of preparing a compound of the formula defined in claim 1, which comprises reacting 3,5-dichloro-2(p-hydroxyphenocy)pyridine with a propionic acid derivative CH₃CHXCR¹ in which more than 50% by ⁰ weight of the propionic acid derivative is in the L optical isomer form, and wherein X is bromine or chlorine, in the presence of a base and a solvent or diluent for the reactants, and recovering a compound of the formula (II) as defined in claim 1.
